# EUROPÄISCHE PATENTSCHRIFT

(11) **EP 0 824 688 B1**
(45) Veröffentlichungstag und Bekanntmachung des Hinweises auf die Patenterteilung: **06.02.2002**
(21) Anmeldenummer: 96914935.0
(22) Anmeldetag: 18.04.1996
(51) Int. Cl.: G01N 27/12, B60H 3/00

(54) **SENSORANORDNUNG ZUR STEUERUNG DER BELÜFTUNG VON INNENRÄUMEN**
SENSOR ASSEMBLY FOR CONTROLLING THE VENTILATION OF INDOOR SPACES
ENSEMBLE DETECTEUR POUR LA COMMANDE DE L'AERATION D'ESPACES INTERIEURS

(30) Priorität: 29.04.1995 DE 19515886
(43) Veröffentlichungstag der Anmeldung: 25.02.1998
(73) Patentinhaber: paragon AG, 33129 Delbrück (DE)
(72) Erfinder: RUMP, Hanns, D-63840 Hausen (DE)
(74) Vertreter: Spalthoff, Adolf, Dipl.-Ing.
(86) Internationale Anmeldenummer: EP9601609
(87) Internationale Veröffentlichungsnummer: WO9635115

(56) Entgegenhaltungen:
- WO-A-88/02704
- US-A- 5 320 577
- PATENT ABSTRACTS OF JAPAN vol. 14, no. 349 (P-1084), 27.Juli 1990 & JP,A,02 126146 (FIGARO ENG INC), 15.Mai 1990,

## Beschreibung

Es ist vorgeschlagen worden, die Belüftung von Fahrzeugen derartig zu steuern, daß immer dann, wenn das Fahrzeug eine Zone erhöhter Schadstoffkonzentration durchfährt, die Lüftung von direkter Außenluft auf Umluftbetrieb umgeschaltet wird.

Eine geeignete Sensorik bestimmt dabei den Schadstoffgehalt der Außenluft und wirkt auf die Umluftklappe im oben geschilderten Sinn ein.

Siehe auch:
DE 33 04 324 A1
WO 88/02704
DE 37 31 754 A1
DE 35 26 462 C2
DE 36 65 044 D
EP 0 221 971

Weil übliche Systeme mit einem einzigen Sensorelement, z.B. Zinndioxidsensoren, nur die in Benzin-Abgasen enthaltenen oxidierbaren Gase (Kohlenmonoxid, Kohlenwasserstoffe) detektieren können, wird die Überempfindlichkeit gegenüber Dieselabgasen (Stickoxide, Schwefeldioxid, Geruchsstoffe) bemängelt.

Aus diesem Grunde sind z.B. in der US-A-5 320 577 Systeme mit zwei Sensoren vorgeschlagen worden. Dabei ist ein Sensor für Dieselabgase (z.B. Wolframtrioxid) und ein anderer Sensor für Benzinabgase (z.B. Zinndioxid) vorgesehen. Jedem Sensor ist ein eigener Signalpfad zugeordnet. Zumindestens ist jedem Sensor ein Analog/Digitalwandler zugeordnet, der die Signale an einen zentralen Auswertebaustein weitergibt (z.B. ein Mikroprozessor).

Obwohl die Funktion so aufgebauter Zwei-Sensor-Systeme den Anforderungen entspricht, erzeugt die zweikanalige Signalauswertung relativ hohe Kosten.

Aufgabe dieser Erfindung ist es, durch einkanalige Signalverarbeitung die Kosten und den Aufwand zu senken.

Dabei macht sich die Erfindung folgende Beobachtung zunutze:

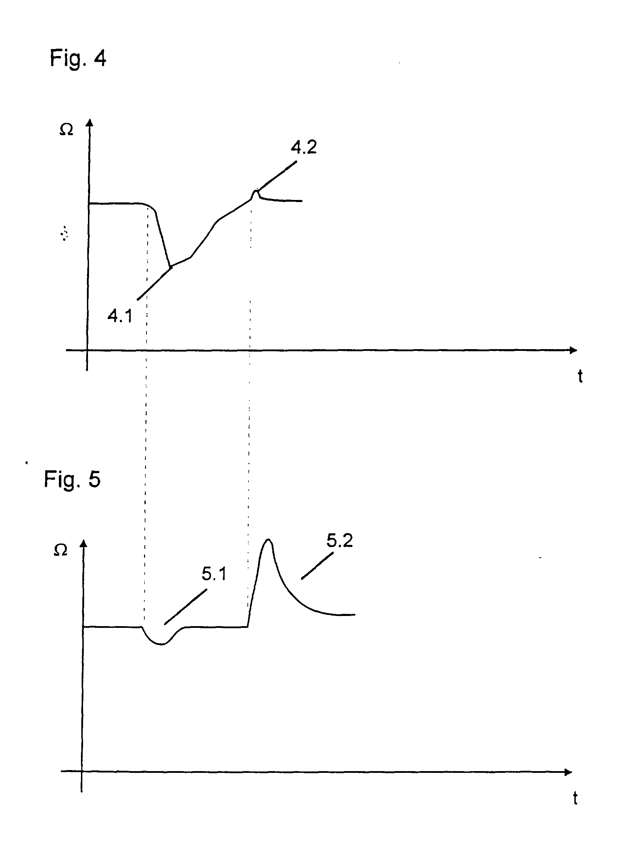

Figur 4 zeigt den typischen Signalverlauf eines Zinndioxid-Sensors, der mit Benzinabgasen 4.1 und später mit Dieselababgasen 4.2 begast wird. Bei Benzinabgasen verringert sich der Sensorwiderstand erheblich. Bei Dieselabgasen erhöht sich der Sensorwiderstand in geringerem Maße.

Figur 5 zeigt den typischen Signalverlauf eines Wolframtrioxid-Sensors, der mit Benzinabgasen 5.1 und später mit Dieselabgasen 5.2 begast wird. Bei Benzinabgasen verringert sich der Sensorwiderstand geringfügig. Bei Dieselabgasen erhöht sich der Sensorwiderstand erheblich.

Die Praxis zeigt, daß beide Gasgruppen fast niemals gleichzeitig in homogener, stöchiometrischer Mischung im Straßenverkehr vorkommen. Vielmehr erreichen eher kurze Gasimpulse verschiedenster Natur den Sensor hintereinander.

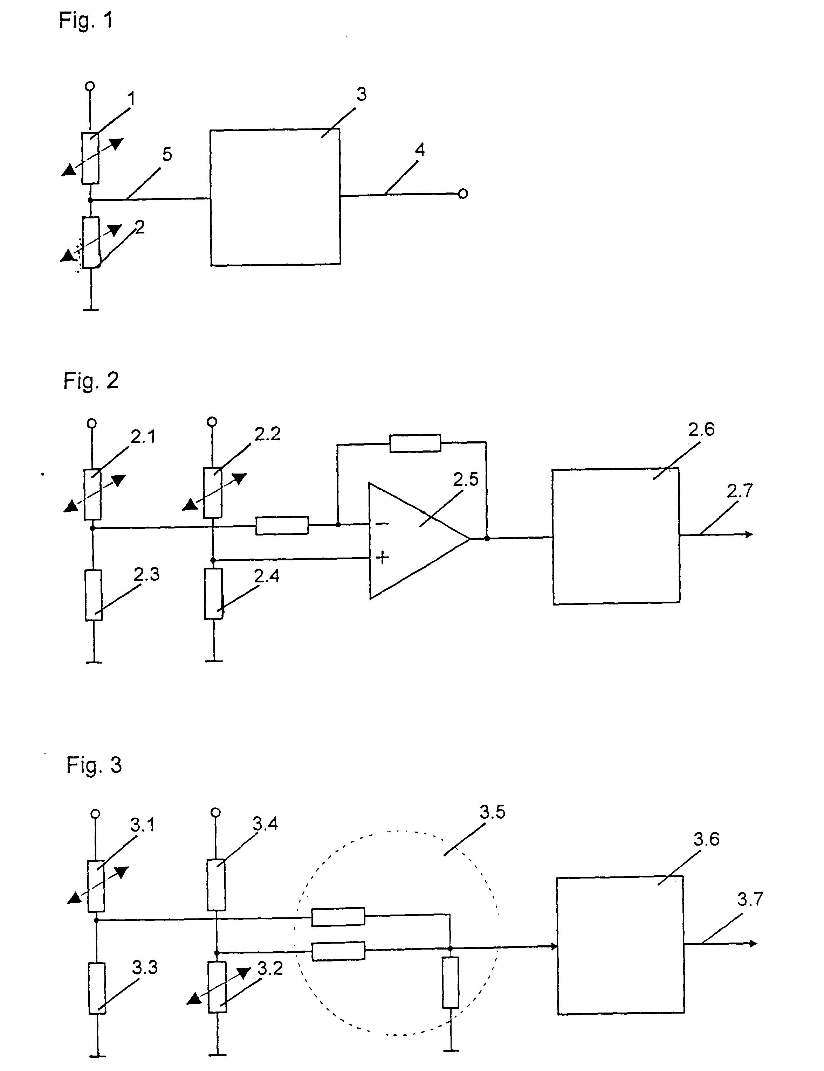

Diese Erfindung schlägt daher einen prinzipiellen Aufbau entsprechend Figur 1 vor. Ein Zinndioxidsensor 1 ist als Spannungsteiler mit einem Wolframtrioxidsensor 2 geschaltet. Die Teilerspannung 5 wird einer Signalverarbeitung 3 zugeführt. Das Ausgangssignal 4 steuert die Umluftklappe. Weil die Diesel- oder Benzin-Abgasimpulse die Sensoren quasi seriell erreichen, wird entweder Sensor 1 niederohmig (Benzinabgase) oder Sensor 2 wird hochohmig, weil Dieselabgase vorliegen. In jedem Fall kann ein in diesem Fall positiver Spannungsimpuls an der Teilerspannung beobachtet werden. Werden die Sensoren miteinander vertauscht, wird selbstverständlich ein negativ gerichteter Spannungsimpuls beobachtet.

Vorteilhaft wird die Teilerspannung in nur einem einzigen Kanal weiterverarbeitet, was den Aufwand erheblich reduziert.

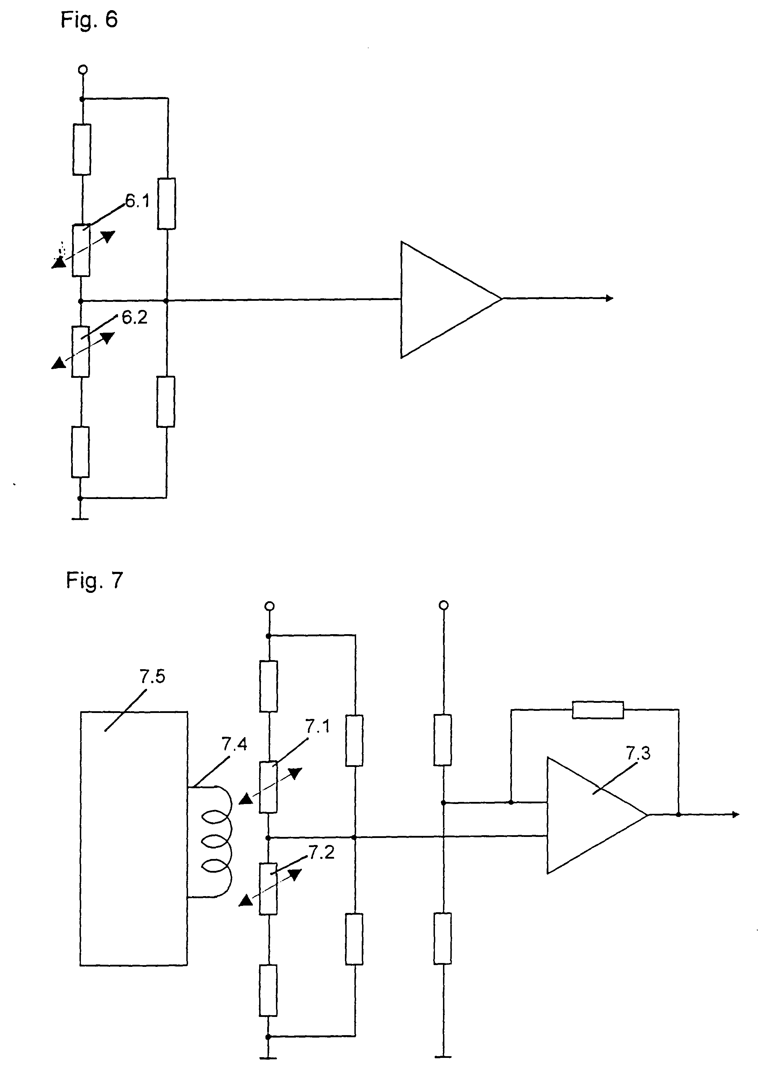

Um die unterschiedlichen Sensorcharakteristiken den physiologischen Erfordernissen anzupassen, wird jedes Sensorelement entsprechend Figur 6 mit einem Reihen- und einem Parallelwiderstand beschaltet. Damit wird erreicht, daß Dieselabgase bei gleicher Angebotskonzentration einen um den Faktor 20-40 höheren Spannungsimpuls erzeugen.

Figur 2 zeigt eine Variante, bei der die Sensoren jeweils mit einem ohmschen Widerstand einen Spannungsteiler bilden. Die Teilerspannungen werden in einem Operationsverstärker 2.5 phasenrichtig addiert, so daß am Ausgang ein gasabhängiger Spannungsimpuls beobachtet wird, der stets in die gleiche Richtung geht, unabhängig von der Natur des angebotenen Gases.

Figur 3 zeigt eine weitere Variante, bei der die Sensoren jeweils im oberen oder unteren Zweig des Spannungsteilers angeordnet sind. Die beiden Teilerspannungen werden addiert 3.5.

Es sind weitere Varianten vorstellbar, wobei stets die beiden Sensoren so geschaltet sind, daß die Tendenzen des Widerstandsverlaufes der beiden Sensoren zur selben Richtung des Spannungsverlaufes führen.

Besonders vorteilhaft kann das Prinzip dieser Erfindung dann eingesetzt werden, wenn Sensorelement und Signalverarbeitung voneinander getrennt werden müssen. Aus Kostengründen wird oft verlangt, daß die komplexe Signalverarbeitung im Klimasteuergerät des Fahrzeuges erfolgt, der Sensor dagegen ausgelagert ist.

Dabei wird vorteilhaft auf einem einzigen Substrat mit einem gemeinsamen Heizer sowohl ein Zinndioxid-Sensor und ein Wolframtrioxidsensor aufgebracht.

Dieser Kombi-Sensor wird nach Figur 7 in eine Schaltung integriert, bei welchem die Teilerspannung eine Impendanzwandlung in einem Operationsverstärker 7.3 erfährt. Eine Reglerelektronik 7.4 speist den Heizwiderstand 7.5 so, daß eine konstante Temperatur unabhängig von Fahrtwind und Außentemperatur erreicht wird.

Das zusammengefaßte Signal beider Sensoren wird über eine einzige elektrische Leitung der Auswertung zugeführt.

Selbstverständlich kann unter Nutzen der erfindungsgemäßen Idee das gleiche Ziel mit zwei einzelnen Sensoren erreicht werden.

Obwohl im Text nur die sensitiven Metalloxide Zinnoxid und Wolframtrioxid erwähnt werden, sind selbstverständlich andere Metalloxide ebenfalls einsetzbar, wie z.B. ZnO, SnxMyOz, Tix-My, Oz, In203, ITO etc. einsetzbar, wobei stets die auf die beiden Gasgruppen empfindlichen Sensoren so beschaltet werden, daß ein in die gleiche Richtung weisender Spannungs- oder Widerstandsverlauf entsteht, der entsprechend in einer Signalverarbeitungsstufe weiterverarbeitet wird.

Es ist dem Fachmann naheliegend, daß für jeden Sensor ein Analogwandler die Sensorwerte digitalisieren kann. Dies erzeugt bei Microcontrollern, die über einen integrierten A/D-Wandler verfügen, keine Mehrkosten. Die Lehre der hier vorgelegten Erfindung gilt auch dann, wenn in der weiteren numerischen Signalverarbeitung die Sensorwerte in der vorgestellten Form zusammengefügt werden.

## Patentansprüche

1. Apparat zur Steuerung der Belüftung von Kabinen, insbesondere von Fahrzeugen, wobei zwei Sensoren (2, 1) zur Detektion von Diesel- und von Benzinabgasen eingesetzt werden, deren Sensorwiderstände sich in entgegengesetzte Richtungen ändern, wenn sie mit Diesel- oder Benzinabgasen beaufschlagt werden, und deren Signale von einer elektronischen Auswerteschaltung (3) ausgewertet werden, derart, daß die direkte Zuführung von Außenluft dann unterbrochen wird und auf Umluftbetrieb umgeschaltet wird, wenn die Außenluftqualität sich verschlechtert, **dadurch gekennzeichnet, daß** der für die Detektion von Dieselabgasen eingesetzte Sensor (2) und der für die Detektion von Benzinabgasen eingesetzte Sensor (1) vor der Auswerteschaltung (3) elektrisch so zu einem einzigen Signalkanal zusammengeschaltet sind und daß die bei der Detektion von Diesel- oder Benzinabgasen ausgelöste Änderung des entsprechenden Sensorwiderstandes zu einer entsprechenden Veränderung des elektrischen Signals im Signalkanal führt, wobei die Signalveränderung unabhängig von der Natur des Gases den gleichen Richtungsverlauf hat.

2. Apparat nach Anspruch 1, **dadurch gekennzeichnet, daß** ein zur Detektion von Dieselabgasen eingerichteter Sensor (2) in Reihe geschaltet wird mit einem zur Detektion von Benzinabgasen eingerichteten Sensor (1) und ein elektrisches Signal (5) zwischen den Sensoren (2, 1) abgegriffen wird.

3. Apparat nach Anspruch 1, **dadurch gekennzeichnet, daß** die für die Detektion von Benzin- oder Dieselabgasen eingerichteten Sensoren (2.1, 2.2) jeweils mit einem ohmschen Widerstand (2.3, 2.4) zu einem Spannungsteiler zusammengeschaltet sind, wobei die zwischen dem Sensor (2.1, 2.2) und dem ohmschen Widerstand (2.3, 2.4) abgegriffenen elektrischen Signale voneinander substrahiert werden.

4. Apparat nach Anspruch 1, **dadurch gekennzeichnet, daß** die für die Detektion von Benzin- oder Dieselabgasen eingerichteten Sensoren (3.1, 3.2) jeweils mit einem ohmschen Widerstand (3.3, 3.4) zu einem Spannungsteiler zusammengeschaltet sind, wobei die zwischen dem Sensor (2.1, 2.2) und dem ohmschen Widerstand (2.3, 2.4) abgegriffenen elektrischen Signale miteinander addiert werden.

5. Apparat nach Anspruch 1 bis 4, **dadurch gekennzeichnet, daß** die beiden Sensoren (6.1, 6.2) jeweils mit einem Widerstand in Reihe geschaltet sind und zu den beiden Reihenschaltungen aus Sensor (6.1, 6.2) und Widerstand jeweils ein weiterer Widerstand parallel geschaltet ist.

## Claims

1. Apparatus for controlling the ventilation of passenger compartments, especially of vehicles, two sensors (2, 1) being used to detect diesel and petrol exhaust gases, their sensor resistances changing in opposite directions when they are acted upon by diesel or petrol exhaust gases and their signals being evaluated by an electronic evaluation circuit (3) in such a manner that the direct supply of external air is interrupted and a switch-over is effected to recirculating air operation when the quality of the external air deteriorates, **characterised in that** the sensor (2) used to detect diesel exhaust gases and the sensor (1) used to detect petrol exhaust gases are interconnected electrically upstream of the evaluation circuit (3) to form a single signal channel in such a manner that the change in the corresponding sensor resistance triggered when diesel or petrol exhaust gases are detected leads to a corresponding change in the electrical signal in the signal channel, the signal change having the same direction characteristic irrespective of the nature of the gas.

2. Apparatus according to claim 1, **characterised in that** a sensor (2) set up to detect diesel exhaust gases is connected in series with a sensor (1) set up to detect petrol exhaust gases, and an electrical signal (5) is tapped between the sensors (2, 1).

3. Apparatus according to claim 1, **characterised in that** each of the sensors (2.1, 2.2) set up to detect petrol or diesel exhaust gases is interconnected with a respective ohmic resistor (2.3, 2.4) to form a voltage divider, the electrical signals tapped between the sensor (2.1, 2.2) and the ohmic resistor (2.3, 2.4) being subtracted from one another.

4. Apparatus according to claim 1, **characterised in that** each of the sensors (3.1, 3.2) set up to detect petrol or diesel exhaust gases is interconnected with a respective ohmic resistor (3.3, 3.4) to form a voltage divider, the electrical signals tapped between the sensor (2.1, 2.2) and the ohmic resistor (2.3, 2.4) being added to one another.

5. Apparatus according to claims 1 to 4, **characterised in that** each of the two sensors (6.1, 6.2) is connected in series with a respective resistance and a respective further resistance is connected in parallel with each of the two series connections comprising a sensor (6.1, 6.2) and a resistance.

## Revendications

1. Appareil pour la commande de l'aération de cabines, en particulier de véhicules, utilisant pour la détection de gaz d'échappement diesel et essence deux capteurs (2, 1) dont les résistances varient en sens contraire lorsqu'ils sont sont sollicités par des gaz d'échappement diesel et essence et dont les signaux sont exploités par un circuit électronique de traitement (3) de manière à supprimer l'admission directe d'air extérieur et commuter sur une recirculation de l'air intérieur en cas de dégradation de la qualité de l'air extérieur, **caractérisé par le fait que** le capteur (2) utilisé pour la détection de gaz d'échappement diesel et le capteur (1) utilisé pour la détection de gaz d'échappement essence sont interconnectés électriquement en amont du circuit de traitement (3) en un unique canal de signal de telle manière que la variation de la résistance des capteurs déclenchée par une détection de gaz d'échappement diesel ou essence conduise à une variation correspondante du signal électrique dans le canal de signal, la variation de signal ayant le même sens d'évolution indépendamment de la nature des gaz.

2. Appareil selon la revendication 1, **caractérisé par le fait qu'**un capteur (2) conçu pour la détection de gaz d'échappement diesel est monté en série avec un capteur (1) conçu pour la détection de gaz d'échappement essence et qu'un signal électrique (5) est prélevé entre les capteurs (2, 1).

3. Appareil selon la revendication 1, **caractérisé par le fait que** les capteurs (2.1, 2.2) conçus pour la détection de gaz d'échappement essence ou diesel sont connectés chacun avec une résistance ohmique (2.3, 2.4) en un diviseur de tension, les signaux électriques prélevés entre le capteur (2.1, 2.2) et la résistance ohmique (2.3, 2.4) étant soustraites l'une de l'autre.

4. Appareil selon la revendication 1, **caractérisé par le fait que** les capteurs (3.1,3.2) conçus pour la détection de gaz d'échappement essence ou diesel sont connectés chacun avec une résistance ohmique (3.3, 3.4) en un diviseur de tension, les signaux électriques prélevés entre le capteur 32.1, 3.2) et la résistance ohmique (2.3, 2.4) étant additionnés l'un à l'autre.

5. Appareil selon la revendication 1 à 4, **caractérisé par le fait que** les capteurs (6.1, 6.2) sont montés chacun en série avec une résistance et qu'une résistance supplémentaire est montée en parallèle avec chacun des deux montages série d'un capteur (6.1, 6.2) et d'une résistance.
